# EUROPEAN PATENT SPECIFICATION

(11) **EP 0 879 294 B1**
(45) Date of publication and mention of the grant of the patent: **02.05.2003**
(21) Application number: 97904089.6
(22) Date of filing: 05.02.1997
(51) Int. Cl.: C12N 5/10, C12N 15/861

(54) **TRANSDUCED HUMAN HEMATOPOIETIC STEM CELLS**
TRANSDUKTION VON MENSCHLICHEN HÄMATOPOIESTISCHEN STAMMZELLEN
CELLULES SOUCHES HEMATOPOIETIQUES TRANSDUITES

(30) Priority: 05.02.1996 US 11172 P; 06.02.1996 US 11219 P; 12.07.1996 US 21683 P
(43) Date of publication of application: 25.11.1998
(73) Proprietor: CELL GENESYS, INC., Foster City, CA 94404 (US)
(72) Inventor: JORDAN, Craig, T., Oakland, CA 94611 (US); HARDY, Steve, San Francisco, CA 94118 (US)
(74) Representative: Schüssler, Andrea, Dr.
(86) International application number: US9701476
(87) International publication number: WO97028269

(56) References cited:
- BLOOD, vol. 84, no. 5, 1 August 1994, pages 1492-1500, XP000674235 S. GOODMAN ET AL.: "Recombinant adeno-associated virus-mediated gene transfer into hematopoietic progenitor cells"
- BLOOD, vol. 86, no. 10 supplement 1, 15 November 1995, page 410a XP000674234 H. ZHU ET AL.: "Adeno-associated virus transduced human CD34+ cells express marker genes during in vitro T-lymphopoiesis"
- BLOOD, vol. 84, no. 1, 1 July 1994, pages 53-58, XP000674233 F. LI ET AL.: "CD34+ peripheral blood progenitors as a target for genetic correction of the two flavocytochrome b558 defective forms of chronic granulomatous disease"
- BLOOD, vol. 81, no. 1, 1 January 1993, pages 254-263, XP000579178 EINERHAND M P W ET AL: "Factors affecting the transduction of pluripotent hematopoietic stem cells: Long-term expression of a human adenosine deaminase gene in mice"
- BLOOD, vol. 87, no. 12, 15 June 1996, pages 5032-5039, XP000674236 T. WATANABE ET AL.: "Gene transfer into human bone marrow hematopoietic cells mediated by adenovirus vectors"
- BLOOD, vol. 88, no. 4, 15 August 1996, pages 1147-1155, XP000674228 S.J. NEERING ET AL.: "Transduction of primitive hematopoietic cells with recombinant adenovirus vectors"

## Description

### 1.0. INTRODUCTION

The present invention relates to human cell biology, and discloses a novel subset of CD34⁺ human hematopoietic stem cells defined by their ability to express recombinant genetic material which has been introduced to the cells by any of a variety methods.

### 2.0. BACKGROUND

Many human tissues such as skin, blood, and internal epithelial tissue are composed of relatively short-lived cells. Because these cells are short-lived, the body must constantly regenerate these cells. Stem cells are a special class of cells in the body that may either divide symmetrically to produce two identical stem cells or asymmetrically to produce one new stem cell and one determined or fully differentiated cell (which will replace the short-lived cell). Accordingly, in order to maintain these tissues, the body must ensure that a carefully regulated supply of stem cells is maintained throughout life.

Additionally, stem cells may be totipotent (i.e., germ line stem cells), pluripotent (i.e., CD34⁺ hematopoietic stem cells), or unipotent (i.e., CD10⁺ lymphoid progenitor cells).

During mammalian embryogenesis, hematopoietic stem cells migrate via the bloodstream to the liver and spleen to seed these tissues, which then carry the burden of hematopoiesis until birth and for some time thereafter. Foci of hematopoiesis can been detected in the liver by the sixth week, although in fetal rats the spleen, along with the mesentery and intestine, appears to have a specific affinity for inoculated adult lymphocytes from the earliest gestational age examined (Chen and McCullagh (1992) J. Reprod. Immunol. 22:127-141). Erythropoiesis predominates in the fetal liver and spleen, although some granulopoiesis also occurs. Hematopoiesis in the fetal liver and spleen does not involve synchronous cell growth, and results enucleated blood cells. Synthesized hemoglobin is not limited to the embryonic type.

The fetal spleen transiently serves as a hematopoietic organ between the third and fifth months of gestation. Pre-B cells (CD24⁺, sIgM⁻) can be detected in the human fetal spleen by 12 weeks (Solvason and Kearney (1992) J. Exp. Med. 175: 397-404).

Several studies have begun to elucidate, at a molecular level, some of the processes of cell migration and hematopoietic maturation and differentiation occurring in the fetal spleen. Recombinant human granulocyte colony stimulating factor (rhG-CSF) administered to pregnant rats has been shown to cross the placenta and specifically induce bone marrow and spleen myelopoiesis in the fetus and neonate. The fetal and neonatal spleen displays an exquisite degree of developmental sensitivity to this cytokine, which results in increased white blood cell counts due to circulating numbers of polymorphonuclear (PMN) cells and increasing the number of post-mitotic (PMN, bands, and metamyelocytes) and mitotic (promyeloblasts, myeloblasts, and metamyeloblasts) myeloid cells in the spleens of neonates (Medlock et al. (1993) Blood 81:916-922). The majority of B cells in the fetal human spleen express, *inter alia,* CD5 and CD10 and can be induced to produce IgM, IgG, IgG4, and IgE (but not IgA) in response to IL-4 in the presence of anti-CD40 monoclonal antibody (mAb) or cloned CD4 T cells (Punnonen et al. (1992) J. Immunol. 148:398-404). It has also been demonstrated that fetal splenic mononuclear cells produce IL-2 and IL-6. Levels of IL-2 and IL-6 increase with gestational age and correlate positively with natural killer (NK) cell activities (Lu et al. (1992) Shih Yen Sheng Wu Hsueh Pao (China) 25:305-309).

Eventually, the site of hematopoiesis is transferred to the bone marrow, which is predominantly granulopoietic. Beginning at the second month of fetal development, the bone marrow plays an increasingly important role in hematopoiesis, becoming the predominant site for hematopoiesis by the second half of gestation. After birth, the bone marrow is eventually the only hematopoietic organ, although both the liver and the spleen can serve as sites of extramedullary hematopoiesis if the bone marrow fails. For a recent review of embryonic and fetal hematopoiesis, see Tavassoli (1991) Blood Cells 17:269-281. An interesting feature of developmental hematopoiesis is that CD34⁺ stem cells that are introduced into the bloodstream will eventually "home" to the bone marrow.

Because stem cells are essentially immortal within the body, they constitute a particularly desirable target for human gene therapy. Where the recombinantly encoded product is preferably delivered to the bloodstream, hematopoietic stem cells (e.g., CD34⁺ cells) are particularly attractive targets for gene delivery. Unfortunately, the relatively low number of CD34⁺ hematopoietic stem cells in the body, as well as the inherent inefficiency of several prior transduction systems (i.e., calcium phosphate transfection, etc.) had made the efficient targeting and transfection of CD34⁺ hematopoietic stem cells somewhat difficult. This has proved to be especially true where viral vectors have been employed for gene delivery because of low inherent infectabilty or the presence of toxic contaminants within the viral preparations, and the inherent toxicity that is often associated with the exposure of cells to high titer virus stocks.

An additional consideration is that some viral vectors require that the target cells are either actively replicating, or at a given stage of the cell cycle, in order to efficiently express the recombinant genetic material of interest. Moreover, the presence of certain cellular receptors may also be required for the efficient infection and delivery of genetic material of interest to hematopoietic stem cells.

An additional consideration when targeting CD34⁺ hematopoietic stem cells for gene delivery is that the expression of the introduced recombinant genetic material may prove somewhat variable. The presently observed differential expression of introduced genetic material within the CD34⁺ cell population defines a new subgroup, or tropism, within the population which may prove particularly important for gene delivery applications.

Adenovirus have proved to be of particular interest for the viral transduction of stem cells because of several features of adenoviral biology (See generally, Berkner, K.L. (1992) Curr. Top. Microbiol. Immunol. 158:39-66). For instance, viral concentration, or titer, is often an important factor in achieving high efficiency transduction of mammalian cells. Adenovirus, by virtue of their life-style, generally allow growth conditions which result in production of higher titer stocks then other mammalian virus.

Also unlike other viruses, adenovirus capsids are not enveloped. Because of this fact, adenovirus particles are quite stable, and may retain infectivity after any of a variety of laboratory procedures. Procedures of particular interest include methods of concentrating infective virus, e.g., CsCl centrifugation, or methods that allow virus to be stored for relatively long periods while retaining substantial infectivity.

Furthermore, the expression of genes encoded by recombinant adenovirus does not require target cell proliferation or viral integration, although a small subset of the adenovirus presumably integrate into the host genome during infection. Hence, adenoviral vectors are generally better suited than other viral vectors for the transduction of postmitotic, slowly proliferating, or nonreplicating cells.

Additionally, particularly where species-specific infection is preferred, replication deficient human, or murine, adenovirus are available for the construction of recombinant virus particles that express a gene of interest. Thus, unlike transduction systems using other eucaryotic virus vectors, recombinant adenovirus can be engineered to utilize viral coat proteins which normally facilitate the normal infection of human cells or cells of other species, rather then rely on the viral coats of a less specific, or amphotropic, nature. This species specificity appears to result in more efficient infection kinetics than can generally be obtained by virus with less specific infectivity.

An additional advantage of using adenovirus for gene delivery is that the genetic material transduced (to be expressed) into the host cell is DNA. Thus, expression of the transduced gene does not need to be preceded by reverse transcription. This is particularly advantageous where the intended recipient is undergoing treatment for the suppression of retroviral disease (i.e., AZT treatment to inhibit reverse transcriptase activity), such as treatment for acquired immunodeficiency syndrome (AIDS).

Recombinant adenoviral vectors have been generated which express a variety of genes. Perhaps most notable is the replication deficient adenovirus vector Ad.RSV that expresses incorporated genetic material of interest using an incorporated promoter from the Rous Sarcoma Virus. In particular, Ad.RSV beta gal (which expresses the bacterial β-galactosidase gene) has been used as a marker for in vivo gene transfer experiments involving salivary glands (Mastrangeli et al. (1994) Am. J. Physiol. 266:1146-1155); mesothelial cells (Setoguchi et al. (1994) Am. J. Respir. Cell. Mol. Biol. 10(4):369-377); and tumor cells (Brody et al. (1994) Hum. Gene Ther. 5(4):437-447, Chen et al. (1994) Proc. Natl. Acad. Sci., U.S.A. 91(8):3054-3057).

In general, adenoviral transduction results in a more transient expression of the inserted genetic material relative to other viral gene delivery systems (i.e., retrovirus, and adeno-associated virus).

Goodman et al. (Blood 84: 1492-1500) and Zhu et al. (Blood 86: Suppl. 1, page 410a) concern the transfer of the β-galactosidase gene in CD34⁺ human bone marrow cells using an adeno-associated virus vector, resulting in in-vitro expression of the β-gal gene.

Einerhand et al. (Blood 81: 254-263) and Li et al. (Blood 84: 53-58) disclose transduction of CD34⁺ human pluripotent haematopoietic stem cells with replication deficient retroviral vectors, resulting in the expression of the adenosine deaminase gene and flavocytochrome genes.

### 3.0. SUMMARY OF THE INVENTION

The subject invention provides for methods and processes for the identification, isolation, and use of a novel subclass of CD34⁺ human hematopoietic stem cells that are capable of being transfected with a chimeric adenovirus to express recombinant genetic material of interest.

Accordingly, an important embodiment of the present invention is a CD34⁺ stem cell that has been virally transduced with a recombinant adenovirus, such that the transduced cell is capable of expressing the transduced genetic material of interest *in vivo*.

Preferably, the titer and infectivity of the transducing virus will be sufficient to allow efficient transduction of the claimed human CD34⁺ cells without requiring a period of selective culture to preferentially expand the number of transduced cells. Accordingly, the transducing virus used in the present invention need not comprise or encode a functional selectable marker.

After being transfected with a vector encoding a suitable gene product, or tagging with an appropriately labeled antibody or receptor specific for the subclass, the novel high-expressing subclass of transduced CD34⁺ hematopoietic stem cells may be sorted and isolated for further manipulation or study using any of a variety of well known techniques including fluorescence activated cell sorting (FACS), centrifugation, or the like.

### 4.0. DESCRIPTION OF THE FIGURES

Figure 1 is a titration curve of alkaline phosphatase expression in the target CD34⁺ cell population as a function of the multiplicity of infection of input recombinant adenovirus.

Figure 2 shows the quasi transient nature of recombinant gene expression after the target CD34⁺ cell population was enzymatically tagged with a recombinant adenovirus comprising the *lacZ* gene. The graph shows the diminution of lacZ activity over 8 days post infection.

Figure 3 compares the levels of alkaline phosphatase expression in populations of: uninfected CD34⁺ cells (Fig. 3A), or CD34⁺ cells that have been infected with a recombinant adenovirus encoding an alkaline phosphatase gene, (Fig. 3B).

Figure 4 compares the levels of alkaline phosphatase expression in populations of quiescent (G₀) CD34⁺ cells using either uninfected CD34⁺ cells (Fig. 4A), or CD34⁺ cells infected with a recombinant adenovirus encoding an alkaline phosphatase gene expressed (Fig. 4B).

### 5.0. DETAILED DESCRIPTION OF THE INVENTION

The present invention allows for the efficient and practical identification and isolation of primary human CD34⁺ stem cells that are permissive for the uptake and expression of genetic material of interest. As used herein, the term "expression" refers to the transcription of the DNA of interest, and the splicing (if any), processing, stability, and, optionally, translation of the corresponding mRNA transcript.

The genetic material of interest can optionally comprise a gene, or fraction thereof, oriented to express either a polypeptide or protein of interest, a "sense" or "antisense" nucleic acid of structural or regulatory importance, or a functional ribozyme. Preferably, such nucleic acid products will be pharmaceutically active or shall provide a therapeutic benefit to the patient.

Preferably, the DNA of interest will be placed in an expression cassette that contains a eucaryotic promoter and/or enhancer region, an insertion site for the genetic material of interest, and a substantially noncoding 3' DNA which facilitates the stability, polyadenlyation, or splicing of the transcript. Any number of transcriptional promoters and enhancers may be used in the expression cassette, including, but not limited to, the herpes simplex thymidine kinase promoter, cytomegalovirus (CMV) promoter/enhancer, SV40 promoters, and retroviral long terminal repeat (LTR) promoter/enhancers. Of special interest are any of a number of well characterized retroviral promoters, particularly the Moloney murine leukemia virus (MLV) LTR promoter and the human immunodeficiency virus (HIV) LTR.

The genetic material of interest will generally constitute nucleic acid which encodes pharmaceutically active products or proteinaceous material. Preferably, the pharmaceutically active protein will provide a therapeutic benefit to the patient. The expressed form of the encoded proteinaceous material may or may not comprise sugar residues. The encoded genetic material of interest may encode a product which is useful in both human or veterinary medicine, either by way of treatment or prophylaxis of diseases or their symptoms, or is useful cosmetically or diagnostically.

Although virtually any DNA sequence of interest may be expressed in CD34⁺ stem cells, particular examples of encoded genetic material of interest which may be used in accordance with this invention include, but are not limited to, protein hormones such as insulin, calcitonin and growth hormone, erythropoietin, plasminogen activators and their precursors, such as t-PA, urokinase, G-CSF, GM-CSF, stem cell factor (SCF) or other cytokines, pro-urokinase and streptokinase, interferons including human interferon alpha, interleukins including IL-1, IL-2, IL-3, IL-4, IL-5, IL-6, IL-7, IL-8, and blood factors including Factor VIII. Additionally, the claimed CD34⁺ cells may be used to express virtually any proteinaceous endocrine hormones as well as any proteinaceous cell or viral receptors.

Given their crucial role in hematopoiesis, the claimed CD34⁺ stem cells may also prove useful as targets for the delivery of genes encoding anti-malarial factors, gene therapy (i.e. adenosine deaminase deficiency, or β-thalassemia, etc.), or, where vectors targeting specific gene replacement are used, as a means of correcting gene defects that are associated with sickle cell anemia.

Optionally, suicide sequences may also be stably and quiescently inserted into stem cells such that the cells will only express the toxic suicide gene upon activation by a specific stimuli. For example, the gene encoding the polio virus translation inhibition protein may placed under-the transcriptional control of the HIV LTR promoter and inserted into the target stem cell. The HIV promoter will remain inactive until the cells are infected with HIV virus which expresses the appropriate trans-acting transcription factors that induce the HIV LTR mediated expression of the suicide product. After the suicide product is expressed, the cells die and the further spread of the virus is effectively eliminated.

Many cytokines and factors are toxic when used systemically. By using transduced stem cells as bioreactors to produce and deliver a protein or peptide of interest to the body, one can effectively deliver local concentrations of factor (in the vicinity of the transduced cells) while effectively maintaining very low systemic concentrations of factor (thus avoiding much of the systemic toxicity effects). This feature of the present invention is particularly important given the fact that infused CD34⁺ stem cells will "home" to the bone marrow. Accordingly, the claimed transduced stem cells may be used to produce and target various therapeutic agents to the bone marrow. Thus, a particularly useful embodiment of the presently disclosed invention is the use of the claimed cells to produce and deliver erythropoietin (or other cytokines) to the bone marrow. Depending on the mode of transduction used, factor production and delivery may be permanent or temporary.

An additional feature of the present mode of factor production and delivery is that one is able to avoid the lengthy purification, formulation, and packaging processes that are typically required where direct introduction of pharmaceutical compositions comprising purified factors is contemplated.

Although enduring, or permanent, expression of the genetic material of interest may often be preferred, there are many instances where transient expression of recombinant genetic material of interest-is more desirable. For example, transient expression may be preferred where one is simply delivering a viral receptor to the stem cells in order the increase or enhance the infectivity of transducing virus that will integrate and stably express a cloned genetic material of interest (e.g., retrovirus or adeno-associated virus).

Additionally, transient transfection and expression may be particularly preferable where acute diseases are involved. For example, by inserting the proper drug resistance factor, transduced CD34⁺ stem cells may be temporarily rendered immune to specific antibiotic or chemotherapeutic agents. Given that the hematopoietic system, is often adversely impacted by the effects of chemotherapeutic treatment, CD34⁺ stem cells may be transduced to transiently express factors that enhance the cells', and surrounding cells', resistance to a given chemotherapeutic agent.

Aplastic anemia is a grave complication that may accompany treatment with a variety of therapeutic agents (i.e., chloramphenicol, *inter alia*). Thus, the introduction of suitable drug resistance genes into CD34⁺ cells, *in vivo* or *in vitro*, may protect these cells from the potentially harmful side-effects of otherwise therapeutic agents.

Similarly, a regulatory gene or antisense nucleic acid may be delivered into the CD34⁺ cell population which transiently and reversibly disrupts stem cell division or DNA synthesis during the period in which the patient is exposed to high doses of chemotherapy or ionizing radiation.

The terms substantially arresting or substantially inhibiting DNA synthesis shall generally mean that the net level/amount of DNA synthesis in treated cells be at least about 70 percent that of control or untreated cells, and preferably mean that the level of DNA synthesis shall be about 50 percent that of control cells. Optionally, the extent of DNA synthesis may be calculated on a per viable cell basis, and normalized accordingly.

Similarly, the use of transient transduction to reversibly inhibit DNA synthesis may effectively render the growth of the target cell population substantially synchronous. For the purposes of this application, the term substantially synchronous population of cells shall mean that generally at least about 50 percent more of the cells in a given cell population will be at or in the same stage of cell division at a given point of interest; preferably at least about 75 percent more of the cells in a given population are at or in the same stage of cell division; and optimally at least about 100 percent more of the cells in a given population will be at or in the same stage of cell division as compared to untreated control cells.

One of ordinary skill will appreciate that, from a medical practitioner's or patient's perspective, virtually any alleviation or prevention of an undesirable symptom (e.g., symptoms related to disease, sensitivity to environmental or factors, normal aging, and the like) would be desirable. Thus, for the purposes of this Application, the terms "treatment", "therapeutic use", or "medicinal use" used herein shall refer to any and all uses of the claimed cells which remedy a disease state or symptoms, or otherwise prevent, hinder, retard, or reverse the progression of disease or other undesirable symptoms in any way whatsoever.

When used in the therapeutic treatment of disease, an appropriate dosage of transducing virus (when targeting CD34⁺ cells *in vivo*) or *in vitro* transduced stem cells or derivatives thereof, may be determined by any of several well established methodologies. Where toxicity is a concern, animal studies are commonly used to determine the maximal tolerable dose, or MTD, of bioactive agent per kilogram weight. In general, at least one of the animal species tested is mammalian. Those skilled in the art regularly extrapolate doses for efficacy and avoiding toxicity to other species, including human. Before human studies of efficacy are undertaken, Phase I clinical studies in normal subjects will help establish safe doses. Preferably, the transducing virus will be prepared such that it is substantially nontoxic to the target cells at high multiplicities of infection (m.o.i., generally exceeding about 100, often exceeding 250, and preferably exceeding about 500 viral particles per CD34⁺ target cell with at least about 50 percent, and preferably at least about 80 percent of the target cells (and/or supporting stromal cells) remaining viable after exposure to the transducing virus).

Where diagnostic, therapeutic or medicinal use of the transduced stem cells, or derivatives thereof, is contemplated, that transduced stem cells to be reimplanted *in vivo* will generally be tested for sterility (absence of mycoplasma, bacteria, fungus, or other potential pathogens), viability, expression of the gene of interest, and for the presence of recombinant viral sequence, and absence of replication competent helper virus. Gene modified stem cells may be introduced *in vivo* by any of a number of established methods, but preferably by intravenous (I.V.) infusion.

Given that the presence of contaminating helper virus genomes may result in the production of replication competent viral particles *in vivo,* the chimeric viral particles used to infect the cells intended to provide a therapeutic benefit *in vivo* will be substantially helper free. For the purposes of this application, the term substantially helper free shall mean that the supernatants from target cells infected with 1 ml undiluted preparation of a given chimeric virus preparation shall not contain significant levels of replication competent virus as identified by plaque assays (i.e., typically less then about ten percent of the infectious plaque activity of comparative titers of replication competent virus). Alternatively, the viral vectors and/or helper-virus may be engineered to incorporate a latent suicide gene (e.g., herpes simplex virus thymidine kinase) that may be activated to kill cells harboring the recombinant virus).

Although the use of chimeric adenovirus similar to those described in WO 96/03163, filed September 23, 1994, are used in the examples, it is contemplated that additional vectors/methods that may be used to deliver nucleotide sequences to the patient including, but are not limited to, liposomal or lipid-associated delivery, direct injection of nucleotides encoding the desired products, and the like.

Optionally, human primary hematopoietic cells will be transduced soon after isolation, and the claimed methods will not require that the primary target cells be adapted to growth in myeloid long-term culture (LTC), nor generally require long periods of culture *in vitro.* Additionally, the claimed methods may not require the use nor the support of fibroblast stromal or "feeder cells" during the transduction procedure, nor to effect or assess expression of the transduced genetic material.

The methods used to culture the stem cells prior to transduction may dramatically effect transduction efficiency. In particular, the presence of cytokines, or combinations thereof, is deemed to be important in preconditioning the stem cells to allow efficient transduction and expression. The cytokines or factors employed during stem cell culture may be of natural, recombinant, or synthetic origin. Although factors derived from other species may be used, factors of human origin are preferred. Examples of such cytokines or factors include, but are not limited to, LIF, interleukins (including IL-1 through IL-14, and analogues thereof), steel factor, colony stimulating factors (CSF), GM-CSF, G-CSF, MIP-1α, Flt-3 ligand, and the like.

Typically, such cytokines and growth factors will be used at concentrations that are amenable to enhancing transduction while not unduly toxic to the CD34⁺ stem cells. Significantly, the presently described cytokines have proven capable of enhancing adenoviral transduction when used at concentrations well below those typically required to stimulate cell growth. Although the amount of cytokine to be added to the culture will generally be cytokine-specific, typically cytokine will be added to the stem cell culture medium an a concentration of between about 1 ng/ml and about 100 ng/ml, generally between about 5 ng/ml and about 50 ng/ml, and preferably between about 5 and about 30 ng/ml.

Generally, where IL-3 is added to the culture to enhance adenoviral transduction it is present at a concentration ranging between about 0.5 ng/ml and about 20 ng/ml, and preferably between about 1 ng/ml and about 5 ng/ml. Generally, where IL-6 is used during stem cell culture it present at a concentration ranging between about 1 ng/ml and about 40 ng/ml, and preferably between about 2 ng/ml and about 10 ng/ml.

Similarly, where SCF is added the culture it is used a concentration ranging between about 2 ng/ml and about 100 ng/ml, and preferably between about 5 ng/ml and about 25 ng/ml.

Where all three of IL-3, IL-6, and SCF are used to provide a synergistic enhancement of adenoviral transduction of CD34⁺ stem cells, the cytokines are typically used at a respective ratio of about 1:2:5, or any of the ranges consistent with the preferred dose ranges for each cytokine as provided above.

Where the virally transduced cells are to be used *in vivo*, the transducing recombinant virus, in addition to being substantially helper free, may optionally be substantially nonimmunogenic (i.e., does not express viral or other immunogenic antigens to such an extent that a substantial immune response against the transduced cells shall be generated *in vivo.*

Regardless of the specific means used to deliver the recombinant nucleic acid of interest to the claimed transduced CD34⁺ cells, the method of gene delivery will preferably be sufficiently efficient that a substantial percentage of transduced CD34⁺ cells may be obtained under nonselective conditions. For the purposes of the present disclosure, the term "a substantial percentage of transduced cells" shall generally mean that at least about five percent of the net amount of CD34⁺ cells initially exposed to the recombinant genetic material will be transduced to express the genes of interest, preferably at least about twenty percent of the CD34⁺ cells exposed to the recombinant genetic material will be transduced to express the genes of interest, specifically at least about thirty percent of the CD34⁺ cells exposed to the recombinant genetic material will be transduced to express the genes of interest. Given the relatively high efficiency envisioned, the vectors used to deliver and guide the expression of the genetic material of interest may contain a selectable marker, but a selectable marker should not required to effect transduction (i.e., the vectors may optionally lack a selectable marker).

The examples below are provided to illustrate the subject invention. These examples are provided by way of illustration and are not included for the purpose of limiting the invention in any way whatsoever.

### 6.0. EXAMPLES

### 6.1. The Transduction of CD34⁺ Stem Cells

A recombinant adenoviral construct (AD-AP) that expresses the alkaline phosphatase gene under the transcriptional control of the Moloney murine leukemia virus (MLV) long terminal repeat (LTR) was used to generate a stock of high-titer virus by standard techniques (Graham and Prevec, 1991, Methods Mol. Biol., 7:109-128). Typically, virus stocks were purified and concentrated by, *inter alia,* CsCl centrifugation (followed by dialysis). The above methods enable the production of adenoviral stocks of low inherent toxicity with titers of at least about 1 x 10¹⁰/ml up to about 10¹³/ml.

Primary human CD34⁺ cells were isolated from human bone marrow, mobilized peripheral blood, or umbilical cord blood using standard procedures (Cellpro Ceprate Kit, or Miltenyi Biotech MACS column). CD34⁺ cells were cultured in X-vivo 10 medium (Biowhitaker) in the presence of one percent human serum albumin, IL-3 (5 ng/ml), IL-6 (10 mg/ml), SCF (25 ng/ml), and five percent fetal bovine serum.

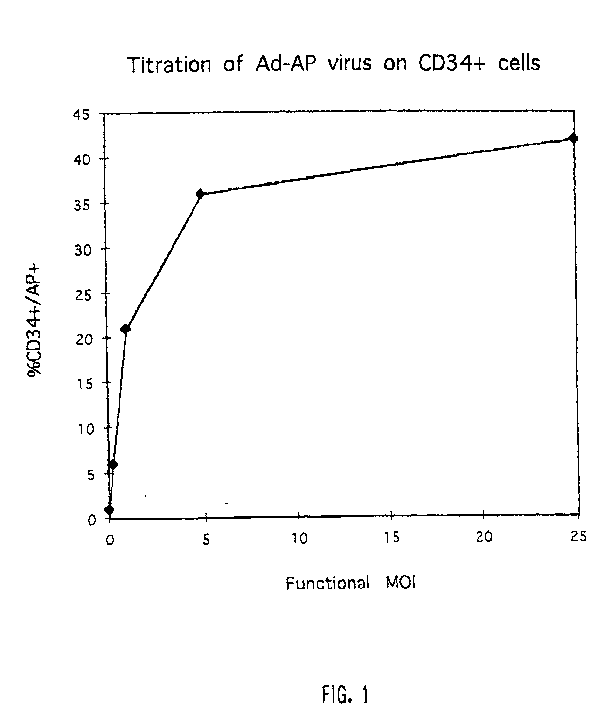

The isolated cells were then infected with the isolated virus at varying multiplicities of infection. The data obtained from this experiment is shown in Figure 1. Figure 1 clearly indicates that the percentage of virally transduced CD34⁺ cells (as measured by detectable alkaline phosphatase expression) increases as increasing amounts of virus are added to the cells. Interestingly, the percentage of transduced cells seems to plateau at about 40 percent.

This observation was repeatable and indicates that only a discrete subset of the CD34⁺ cell population is able to be transduced to express cloned genetic material introduced by the chimeric adenoviral vectors used in the study.

The fact that only a fixed subset of CD34⁺ cells were able to be transduced by adenoviral vectors may be explained by the fact that only a fixed percentage of the stem cells were properly conditioned in culture to be rendered transducible by adenovirus vectors. For example, in order to achieve maximal levels of transfection efficiency, the presently described human CD34⁺ cells must generally be exposed to human cytokines. In particular, the presence of IL-3, IL-6, and stem cell factor (SCF), rapidly increases transfection efficiency, and may even be deemed as essential for the transfection of CD34⁺ stem cells. Each of these cytokines enhance adenovirus transfection when used individually, and, when combined, provide a synergistic effect that typically enhances transfection efficiency by about 25 to about 50 percent greater beyond the mere additive effect of the individual cytokines.

The discovery that cytokine exposure enhances the transfectability of human CD34⁺ stem cells has far reaching application. For example, it is likely that exposure to other cytokines, and particularly mixtures thereof, will render higher percentages of a CD34⁺ population transducible by adenoviral vectors. Additionally, by using the proper cytokines, or synergistic mixtures thereof, to similarly condition a population of CD34⁺ cells, it is likely that the cells will also be rendered more transducible by other means such as retroviral vectors, adenoassociated virus vectors, lipofection, electroporation, etc.

### 6.2. Transient Transduction of CD34⁺ Stem Cells With AD-Lacz

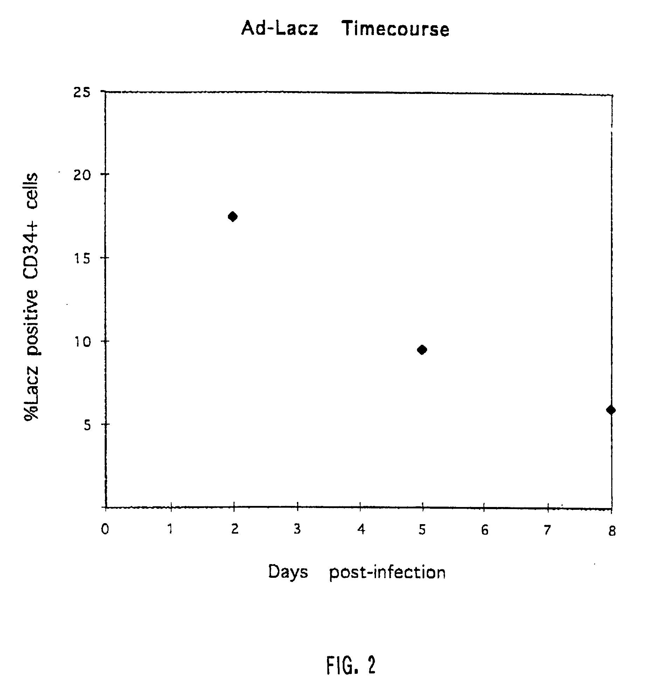

A different adenoviral construct (AD-Lacz) that expresses the *lacZ* gene was constructed and used in a time course experiment that tracked the LacZ expression by the transduced CD34⁺ cells over the course of eight days post-infection. The data resulting from this experiment is shown in Figure 2 which shows that the percentage of transduced cells that expressed LacZ gradually decreased after infection. Interestingly, the decrease was nonlinear and may indicate that although the majority of the target cells are presumably transiently infected, a portion of the target cells may continue to express the introduced genetic material of interest for substantial periods. The nonlinearity of the decrease in expression may also be a function of the gradual dilution of the replication deficient adenoviral encoded sequences as the population of CD34⁺ cells gradually increased in the culture.

### 6.3. Human CD34⁺ Stem Cells May be Transfected With AD-AP to Express AP

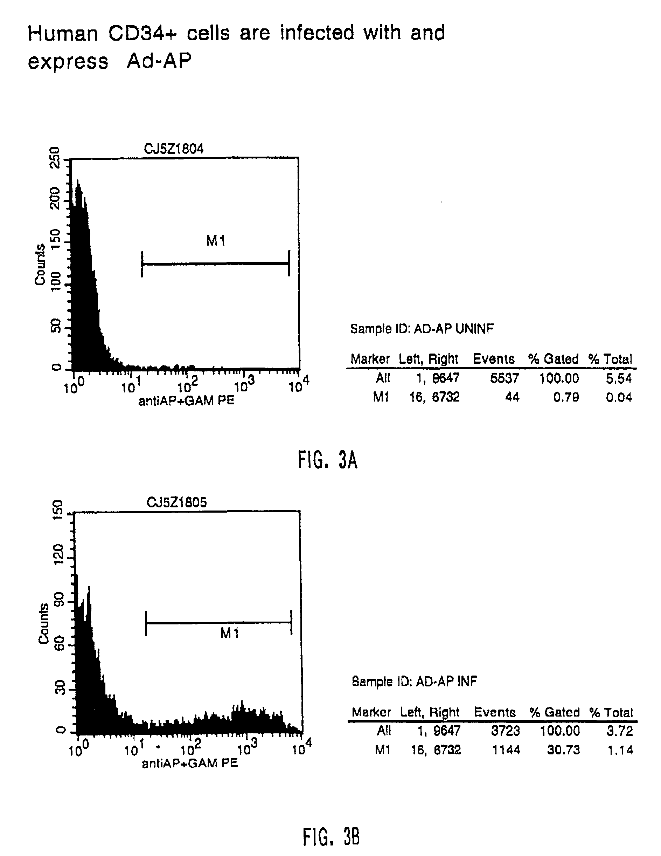

Figure 3A shows the results of a FACS analysis that was used to directly quantify the extent to which uninfected CD34⁺ cells express alkaline phosphatase (AP). In this experiment, the target cells were tagged with mouse anti-AP antibodies, and a fluorescently labeled goat anti-mouse antibody. The data clearly indicate that very little AP expression could be detected in uninfected cells.

Conversely, the FACS analysis shown in Figure 3B clearly indicates that, after infection with AD-AP, a significant percentage of the CD34⁺ cell population expresses the AP gene.

### 6.4. Quiescent Human CD34⁺ Stem Cells May be Transfected With AD-AP to Express AP

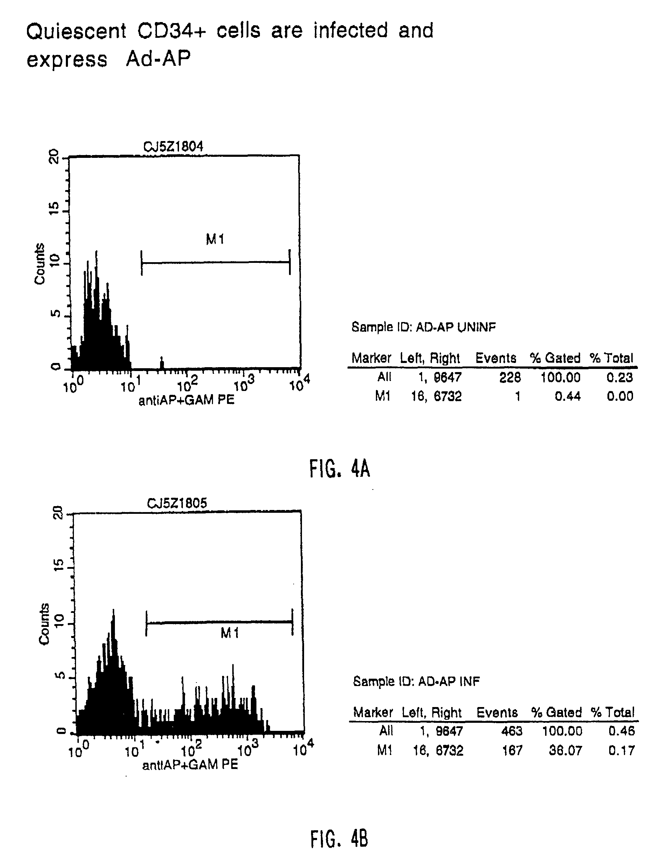

Figure 4A shows the results of a FACS analysis that was used to directly quantify the extent to which uninfected quiescent CD34⁺ cells express alkaline phosphatase (AP). In this experiment, the target cells were tagged with mouse anti-AP antibodies, and a fluorescently labeled goat anti-mouse antibody. The data clearly indicate that very little AP expression could be detected in uninfected cells.

Conversely, the FACS run shown in Figure 4B clearly indicates that, after infection with AD-AP, a significant percentage of the quiescent CD34⁺ cell population expresses the AP gene.

Quiescent CD34⁺ cells were collected based on two separate criteria. First, cells which did not express the Ki-67 antigen, a well known marker of cell activation (Schuler *et al*., 1993, J. Cell Biol. *123(3)*:513-522). In addition, cells were further selected which showed no evidence of DNA replication when tested by staining with 7-amino-actinomycin-D (Rabinovitch *et al.,* 1986, J. Immunol. *136*:2769).

## Claims

1. Transduced human CD34⁺ hematopoietic stem cells comprising recombinant genetic material of interest, said cells having been transduced without a period of selective culture and having the property of being capable of expressing the genetic material of interest, wherein said cells have been transduced by a replication deficient adenovirus comprising the genetic material of interest.

2. Transduced cells according claim 1 wherein said cells express a recombinantly encoded cytokine.

3. Transduced cells according to claim 2, wherein said cytokine is selected from the group consisting of Stem Cell Factor, GM-CSF, IL-3, erythropoietin, IL-6 and Flt-3 ligand.

4. Transduced cells according to any preceding claim wherein said cells express the genetic material of interest *in vitro*.

5. Transduced cells according to any of claims 1 to 3 wherein said cells express the genetic material of interest *in vivo*.

6. Transduced cells, according to any preceding claim, for use in a method of medical treatment of a human or animal body.

7. The use of transduced human CD34⁺ hematopoietic stem cells, as defined in any preceding claim, in the manufacture of a medicament for use in a method of medical treatment of a human or animal body.

8. An in vitro method of making transduced human CD34⁺ hematopoeitic stem cells, comprising:
a) culturing said cells in the presence of IL-3, IL-6, and stem cell factor; and
b) infecting said cells with a purified and concentrated replication deficient chimeric adenovirus containing a gene encoding a pharmaceutically active product.

9. The method according to claim 8 comprising the additional step of the expression by the cells of a recombinantly encoded cytokine, preferably selected from the group consisting of Stem Cell Factor GM-CSF, IL-3, erythropoietin, IL-6 and Flt-3 ligand.

10. The method according to claim 9 wherein the expression of recombinantly encoded cytokine takes place *in vitro*.

## Patentansprüche

1. Transduzierte menschliche hämatopoetische CD34⁺-Stammzellen, umfassend interessierendes rekombinantes genetisches Material, wobei die Zellen ohne eine Phase selektiver Kultur transduziert worden sind und die Eigenschaft aufweisen, das interessierende genetische Material exprimieren zu können, wobei die Zellen durch ein replikationsdefektes Adenovirus transduziert worden sind, das das interessierende genetische Material umfasst.

2. Transduzierte Zellen nach Anspruch 1, wobei die Zellen ein rekombinant kodiertes Zytokin exprimieren.

3. Transduzierte Zellen nach Anspruch 2, wobei das Zytokin aus der Gruppe Stammzellenfaktor, GM-CSF, IL-3, Erythropoietin, IL-6 und Flt-3-Ligand ausgewählt sind.

4. Transduzierte Zellen nach einem der vorhergehenden Ansprüche, wobei die Zellen das interessierende genetische Material in vitro exprimieren.

5. Transduzierte Zellen nach einem der Ansprüche 1 bis 3, wobei die Zellen das interessierende genetische Material in vivo exprimieren.

6. Transduzierte Zellen nach einem der vorhergehenden Ansprüche zur Verwendung in einem Verfahren zur medizinischen Behandlung eines menschlichen oder tierischen Körpers.

7. Verwendung von transduzierten menschlichen hämatopoetischen CD34⁺-Stammzellen, wie in einem der vorhergehenden Ansprüche definiert, bei der Herstellung eines Medikaments zur Verwendung in einem Verfahren zur medizinischen Behandlung eines menschlichen oder tierischen Körpers.

8. In-vitro-Verfahren zum Herstellen von transduzierten menschlichen hämatopoetischen CD34⁺-Stammzellen, umfassend:
a) Kultivieren der Zellen in Anwesenheit von IL-3, IL-6 und Stammzellenfaktor; und
b) Infizieren der Zellen mit einem gereinigten und konzentrierten replikationsdefekten chimären Adenovirus, der ein Gen enthält, das für ein pharmazeutisch aktives Produkt kodiert.

9. Verfahren nach Anspruch 8, umfassend den zusätzlichen Schritt der Expression durch die Zellen eines rekombinant kodierten Zytokins, das vorzugsweise aus der Gruppe Stammzellenfaktor GM-CSF, IL-3, Erythropoietin, IL-6 und Flt-3-Ligand ausgewählt ist.

10. Verfahren nach Anspruch 9, wobei die Expression von rekombinant kodiertem Zytokin in vitro stattfindet.

## Revendications

1. Cellules souches hématopoïétiques CD34⁺ transduites comprenant un matériel génétique recombinant d'intérêt, lesdites cellules ayant été transduites sans une période de culture sélective et ayant la propriété d'être capables d'exprimer le matériel génétique d'intérêt, dans lesquelles lesdites cellules ont été transduites par un adénovirus à réplication déficiente comprenant le matériel génétique d'intérêt.

2. Cellules transduites selon la revendication 1, dans lesquelles lesdites cellules expriment une cytokine qui a été codée par recombinaison.

3. Cellules transduites selon la revendication 2, dans lesquelles ladite cytokine est sélectionnée dans le groupe consistant en facteur des cellules souches, GM-CSF, IL-3, érythropoïétine, IL-6 et ligand de Flt-3.

4. Cellules transduites selon une quelconque revendication précédente, dans lesquelles lesdites cellules expriment le matériel génétique d'intérêt *in vitro*.

5. Cellules transduites selon l'une quelconque des revendications 1 à 3, dans lesquelles lesdites cellules expriment le matériel génétique d'intérêt *in vivo*.

6. Cellules transduites, selon une quelconque revendication précédente, pour utilisation dans une méthode de traitement médical d'un corps humain ou animal.

7. Utilisation de cellules souches hématopoiétiques CD34⁺ transduites, telles que définies dans une quelconque revendication précédente, dans la fabrication d'un médicament destiné à être utilisé dans une méthode de traitement médical d'un corps humain ou animal.

8. Méthode *in vitro* pour produire des cellules souches hématopoïétiques CD34⁺ transduites, comprenant :
a) la mise en culture desdites cellules en présence de IL-3, IL-6, et facteur des cellules souches ; et
b) l'infection desdites cellules avec un adénovirus chimère à réplication. déficiente purifié et concentré contenant un gène codant un produit pharmaceutiquement actif.

9. Méthode selon la revendication 8 comprenant l'étape supplémentaire de l'expression par les cellules d'une cytokine qui a été codée par recombinaison, de préférence sélectionnée dans le groupe consistant en facteur des cellules souches, GM-CSF, IL-3, érythropoïétine, IL-6 et ligand de Flt-3.

10. Méthode selon la revendication 9 dans laquelle l'expression de la cytokine qui a été codée par recombinaison a lieu *in vitro*.
